(19) **Europäisches Patentamt / European Patent Office / Office européen des brevets**

(11) **EP 3 389 770 B1**

(12) **FASCICULE DE BREVET EUROPEEN**

(45) Date de publication et mention de la délivrance du brevet:
**26.08.2020 Bulletin 2020/35**

(21) Numéro de dépôt: **16823179.3**

(22) Date de dépôt: **14.12.2016**

(51) Int Cl.:
*A61N 1/368* (2006.01)          *A61N 1/37* (2006.01)
*A61B 5/0452* (2006.01)

(86) Numéro de dépôt international:
**PCT/EP2016/080880**

(87) Numéro de publication internationale:
**WO 2017/102780 (22.06.2017 Gazette 2017/25)**

(54) **DISPOSITIF MÉDICAL IMPLANTABLE ACTIF DE TYPE RESYNCHRONISEUR CARDIAQUE AVEC ADAPTATION DYNAMIQUE DU DÉLAI ATRIOVENTRICULAIRE EN FONCTION D'UN DEGRÉ DE FUSION DÉTECTÉ ET QUANTIFIÉ**

AKTIVE IMPLANTIERBARE MEDIZINISCHE VORRICHTUNG WIE ETWA EIN HERZRESYNCHRONISATOR MIT DYNAMISCHER ANPASSUNG DER ATRIOVENTRIKULÄREN VERZÖGERUNG IN ABHÄNGIGKEIT EINES ERFASSTEN UND QUANTIFIZIERTEN FUSIONSGRADES

ACTIVE IMPLANTABLE MEDICAL DEVICE SUCH AS A CARDIAC RESYNCHRONISER WITH DYNAMIC ADAPTATION OF ATRIOVENTRICULAR DELAY DEPENDING ON A DETECTED AND QUANTIFIED DEGREE OF FUSION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.12.2015 FR 1562739**

(43) Date de publication de la demande:
**24.10.2018 Bulletin 2018/43**

(73) Titulaire: **Sorin CRM SAS**
**92140 Clamart (FR)**

(72) Inventeurs:
• **MILPIED, Paola**
**13008 Marseille (FR)**
• **FEUERSTEIN, Delphine**
**92130 Issy les Moulineaux (FR)**

(74) Mandataire: **Grünecker Patent- und Rechtsanwälte**
**PartG mbB**
**Leopoldstraße 4**
**80802 München (DE)**

(56) Documents cités:
| | |
|---|---|
| **EP-A1- 1 205 214** | **EP-A1- 1 905 480** |
| **EP-A1- 2 311 524** | **EP-A1- 2 324 885** |
| **EP-A1- 2 742 971** | **EP-A1- 2 742 973** |
| **EP-A1- 2 803 385** | **US-B1- 6 832 112** |

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au cœur des impulsions électriques de stimulation en cas de trouble du rythme détecté par le dispositif.

L'invention concerne plus particulièrement les thérapies de resynchronisation, dites "CRT" (*Cardiac Resynchronization Therapy*) ou "BVP" (*Bi-Ventricular Pacing*), consistant à surveiller en continu le rythme cardiaque et délivrer si nécessaire au cœur des impulsions électriques permettant de provoquer une contraction conjointe des ventricules gauche et droit (ou des oreillettes gauche et droite) afin de resynchroniser ces derniers.

Une telle thérapie CRT permet d'optimiser le cycle contraction/relaxation avec un bénéfice direct facilitant le travail du cœur, notamment dans les pathologies d'insuffisance cardiaque où l'on cherche à stabiliser le phénomène de remodelage cardiaque (c'est-à-dire l'ensemble des modifications du cœur engendrées en réponse à une pathologie, et qui est généralement associé à un plus mauvais pronostic), et même de contrer ce phénomène ("remodelage inverse"), avec pour le patient un meilleur pronostic.

L'invention concerne plus précisément, dans ce contexte, la situation dite de "fusion", c'est-à-dire l'application dans une cavité, de façon plus ou moins concomitante à une dépolarisation spontanée produite naturellement par le nœud sinusal (dans le cas de fusion atriale) et/ou par le nœud atrioventriculaire (dans le cas de fusion ventriculaire), d'une stimulation destinée à induire dans cette même cavité une onde de dépolarisation dite "onde évoquée".

En particulier, la thérapie CRT peut être mise en œuvre avec stimulation du seul ventricule gauche, lorsque la conduction atrioventriculaire droite native est préservée. Le ventricule droit se contracte spontanément, et la stimulation du ventricule gauche est contrôlée de manière que ce dernier se contracte en synchronisme avec le ventricule droit. Ceci réduit le besoin d'une stimulation droite, inutile voire délétère.

Dans ce cas, il convient de chercher à favoriser la fusion de la stimulation du ventricule gauche avec la conduction spontanée du ventricule droit.

On notera toutefois que ce cas particulier n'est pas limitatif de l'invention, qui peut également s'appliquer à d'autres situations de stimulation d'une cavité pour se synchroniser sur le rythme spontané de la cavité opposée : par exemple le cas de la resynchronisation des deux oreillettes entre elles, ou celui d'une stimulation du ventricule droit synchronisée sur la contraction du ventricule gauche.

En présence d'une capture complète (c'est-à-dire lorsque la dépolarisation du ventricule gauche ne résulte que de la stimulation, l'onde évoquée précédant toute dépolarisation spontanée), ou si la stimulation engendre une dépolarisation trop proche de la capture complète, le délai atrioventriculaire (DAV ou AVD) devra être allongé pour garantir la fusion et obtenir l'effet mécanique souhaité de resynchronisation des deux ventricules. Diverses techniques de détection d'une situation de fusion ont été déjà proposées, essentiellement pour des dispositifs de stimulation "double chambre" classiques, c'est-à-dire où le dispositif surveille l'activité ventriculaire après un événement auriculaire spontané ou stimulé, et déclenche une stimulation du ventricule droit si aucune activité spontanée ventriculaire n'a été détectée à l'issue d'un certain délai. Cette détection d'une éventuelle fusion est notamment utile dans le contexte d'un "test de capture" consistant, après avoir stimulé une cavité, à détecter dans cette cavité la présence ou non de l'onde évoquée, ceci afin de déterminer si la stimulation a été efficace ou non et adapter si nécessaire le seuil d'efficacité ou "seuil d'entrainement", afin que l'énergie délivrée soit suffisante pour induire de façon certaine une onde évoquée, mais sans être excessive de manière à ne pas obérer la durée de vie du dispositif.

Le EP 2 324 885 A1 (Sorin CRM) décrit une telle technique, avec classification de chaque cycle cardiaque en i) cycle en capture complète, ii) cycle en fusion ou iii) cycle avec perte de capture. Cette technique présente l'inconvénient de fonctionner en "tout-ou-rien" pour ce qui est de la fusion : c'est-à-dire que tout ce qui ne ressemble ni à une capture complète, ni à une perte de capture est considéré, à défaut, comme une situation de fusion. En d'autres termes, il s'agit d'une technique de classification, binaire. Il ne s'agit pas d'une technique de quantification de la fusion qui permettrait d'évaluer sur une échelle le décalage temporel plus ou moins important entre l'onde évoquée et l'onde du rythme sinusal.

Mais ce document n'envisage pas d'appliquer la détection ou la quantification de la fusion au pilotage d'un dispositif CRT.

Le EP 1 205 214 A1 décrit un dispositif médical actif implantable de type "double chambre" comprenant des moyens de détection d'événements auriculaires et ventriculaires, des moyens de stimulation auriculaire, aptes à délivrer une impulsion de stimulation après écoulement d'un délai atrio-ventriculaire programmé consécutif à la détection d'un événement auriculaire et en l'absence de détection d'événement ventriculaire spontané dans ce délai, des moyens de détection de situations de fusion, aptes à analyser une séquence de cycles cardiaques successifs en modifiant le délai atrio-ventriculaire d'un cycle à l'autre, et des moyens pour détecter la présence ou l'absence d'un événement ventriculaire spontané à l'intérieur du délai atrio-ventriculaire ainsi modifié.

Le EP 2 756 865 A1 décrit une autre technique, permettant non seulement de déterminer les situations de fusion mais en plus de quantifier une fusion. Cette technique repose sur l'analyse d'un EGM unipolaire avec comparaison, pour chaque cycle cardiaque, de l'EGM enregistré avec deux signaux de référence, pour produire deux indices de corrélation adaptatifs signés (ASCI), compris entre -1 et +1. Ces indices sont ensuite multipliés pour traduire la ressemblance plus ou moins grande du cycle cardiaque aux références enregistrées, une situation intermédiaire correspondant à une fusion, quantifiée par le produit des deux indices calculés pour le cycle cardiaque.

Toutefois, cette technique ne permet pas toujours de mettre en évidence la fusion, le signal sur la voie unipolaire ne reflétant pas toujours suffisamment certains changements caractéristiques de la morphologie ou du synchronisme temporel produits par une fusion.

On notera d'ailleurs qu'en stimulation double chambre la fusion est considérée comme un phénomène délétère, et inapproprié dans le cas d'un test de capture, situation qui est l'exacte opposée de celle d'un dispositif CRT où une fusion entre stimulation ventriculaire gauche et contraction spontanée du ventricule droit est recherchée et considérée comme bénéfique.

Le but de l'invention est d'appliquer une technique de détermination d'un degré de fusion au pilotage d'un dispositif de resynchronisation CRT implanté, en particulier d'un dispositif CRT pouvant opérer dans un mode de stimulation du seul ventricule gauche, qui permette d'optimiser le fonctionnement de ce dispositif notamment par un ajustement dynamique et automatique du DAV.

Plus précisément, l'invention propose un dispositif comprenant, de manière en elle-même connue d'après le EP 2 324 885 A1 précité : des moyens de stimulation ventriculaire, aptes à produire des impulsions de stimulation destinées à être délivrées au moins au ventricule gauche d'un patient porteur du dispositif ; des moyens de détection d'événements auriculaires et ventriculaires ; et des moyens d'application d'un délai atrioventriculaire, DAV, entre un événement auriculaire détecté ou stimulé et la délivrance d'une impulsion de stimulation ventriculaire.

De façon caractéristique de l'invention, ce dispositif comprend en outre : des moyens de quantification d'un degré de fusion entre la délivrance d'une impulsion de stimulation à une cavité, gauche ou droite, et une contraction spontanée d'une autre cavité, opposée, respectivement droite ou gauche, ces moyens étant aptes à calculer une valeur de taux de fusion comprise entre deux valeurs extrêmes correspondant respectivement à une situation de capture complète et à une situation de dépolarisation spontanée de ladite cavité ; et des moyens d'adaptation dynamique du DAV, aptes à modifier la valeur du DAV appliqué à la délivrance de ladite impulsion de stimulation ventriculaire, en fonction d'une comparaison opérée entre i) la valeur courante du taux de fusion calculée par les moyens quantificateurs, et ii) une valeur cible de taux de fusion.

Selon diverses caractéristiques subsidiaires avantageuses :

- les moyens de quantification d'un degré de fusion sont des moyens de quantification d'un degré de fusion entre la délivrance d'une impulsion de stimulation au ventricule gauche, et une contraction spontanée du ventricule droit, ces moyens étant aptes à calculer une valeur de taux de fusion comprise entre deux valeurs extrêmes correspondant respectivement à une situation de capture complète et à une situation de dépolarisation spontanée du ventricule gauche ;

- les moyens de détection d'événements auriculaires et ventriculaires comprennent des moyens aptes à recueillir au cours d'un même cycle cardiaque, concurremment sur des voies respectives distinctes, au moins deux signaux différents d'électrogramme endocavitaire, EGM, et en dériver au moins deux composantes temporelles distinctes respectives, et les moyens de quantification d'un degré de fusion comprennent des moyens d'analyse du cycle cardiaque courant, comportant : des moyens aptes à combiner les au moins deux composantes temporelles en au moins une caractéristique 2D paramétrique représentative dudit cycle cardiaque, à partir des variations de l'une des composantes temporelles en fonction de l'autre ; et des moyens aptes à opérer une comparaison de la caractéristique 2D du cycle courant avec au moins une caractéristique 2D de référence obtenue antérieurement et mémorisée par le dispositif ;

- les moyens de quantification d'un degré de fusion sont aptes à calculer ladite valeur de taux de fusion sur un nombre donné C de cycles cardiaques, C ≥ 1, avantageusement un nombre variable, fonction d'un niveau d'activité du patient ;

- les moyens d'adaptation dynamique du DAV comprennent des moyens de modification incrémentale du DAV, aptes à opérer : une incrémentation d'au moins un pas du DAV lorsque la valeur courante du taux de fusion a évolué par rapport au(x) cycle(s) précédent(s) dans le sens d'un rapprochement vers ladite situation de capture complète, et une décrémentation d'au moins un pas du DAV lorsque la valeur courante du taux de fusion a évolué par rapport au(x) cycle(s) précédent(s) dans le sens d'un rapprochement vers ladite situation de de contraction spontanée ;

- dans ce dernier cas, ladite incrémentation/décrémentation est avantageusement une incrémentation/décrémentation d'un nombre variable de pas, ledit nombre de pas étant fonction de l'écart entre la valeur courante et la valeur cible du taux de fusion ;

- le dispositif comprend en outre des moyens de comparaison de la valeur de DAV déterminée par les moyens d'adaptation dynamique du DAV avec un seuil limite supérieur, et des moyens de modification sélective du mode de fonctionnement du dispositif en cas de franchissement dudit seuil limite supérieur ;

- les moyens d'adaptation dynamique du DAV comprennent des moyens de limitation de ladite valeur de DAV modifiée à une valeur plancher minimale ;
- le dispositif comprend en outre : des moyens de stimulation auriculaire, sélectivement commutables entre un mode avec stimulation auriculaire et un mode sans stimulation auriculaire ; des moyens de détection et de comptage, dans un premier compteur, des commutations entre modes avec et sans stimulation auriculaire ; des moyens de détection et de comptage, dans un second compteur, des occurrences où la variation de la valeur courante du taux de fusion entre cycles consécutifs excède un seuil prédéterminé ; et des moyens de modification sélective du mode de fonctionnement du dispositif en cas de dépassement par le premier et le second compteur de valeurs de compte respectives prédéterminées ;
- le dispositif comprend en outre : des moyens de stimulation auriculaire, sélectivement commutables entre un mode avec stimulation auriculaire et un mode sans stimulation auriculaire ; des moyens de détection et de comptage, dans un premier compteur, des commutations entre modes avec et sans stimulation auriculaire ; des moyens de détection et de comptage, dans un second compteur, des occurrences où la variation de la valeur courante du taux de fusion entre cycles consécutifs excède un seuil prédéterminé ; et des moyens de modification sélective du mode de fonctionnement du dispositif en cas de dépassement par le premier compteur d'une première valeur de compte prédéterminée, alors que le second compteur reste au-dessous d'une seconde valeur de compte prédéterminée, et *vice versa.*

On va maintenant décrire un exemple de mise en œuvre de la présente invention, en référence aux dessins annexés où les mêmes références désignent d'une figure à l'autre des éléments identiques ou fonctionnellement semblables.

La Figure 1 est une vue générale montrant un dispositif de stimulation CRT avec son générateur et des sondes cardiaques droite et gauche implantées dans le cœur.
La Figure 2 est un exemple de signaux EGM obtenus sur des voies respectivement ventriculaire bipolaire et ventriculaire unipolaire de l'une des sondes de la Figure 1.
La Figure 3 illustre des exemples de signaux recueillis sur une voie bipolaire et sur une voie unipolaire, pour différentes situations correspondant à une capture complète, à une perte totale de capture laissant apparaitre le rythme sinusal normal NSR, et pour un certain nombre de cycles intermédiaires à tester, avec présence éventuelle d'une fusion plus ou moins marquée, pour des DAV intermédiaires entre les deux situations de capture et de perte de capture.
La Figure 4 illustre la manière de combiner entre eux les signaux bipolaire et unipolaire recueillis dans une même cavité ventriculaire pour construire une caractéristique bidimensionnelle de type vectogramme.
La Figure 5 est un exemple de vectogramme échantillonné obtenu pour un cycle cardiaque échantillonné à 128 Hz, avec représentation des vecteurs vitesse en divers points successifs.
La Figure 6 est un schéma de principe illustrant la mise en œuvre d'une technique préférentielle de quantification du degré de fusion.
La Figure 7 est un organigramme simplifié présentant la séquence d'étapes exécutée pour l'analyse d'un cycle courant.
La Figure 8 est une représentation, dans un espace bidimensionnel, des nuages de points correspondant à diverses valeurs de couples de descripteurs relevées, sur un certain nombre de cycles courants, lors de la comparaison avec les deux références correspondant respectivement à une capture et un rythme sinusal.
La Figure 9 est une représentation montrant les variations de la distance entre les points de l'espace de la Figure 8 et l'origine de ce même espace, pour des valeurs croissantes du DAV.
La Figure 10 est un organigramme présentant la séquence d'étapes exécutée lors de l'élaboration préalable des deux références utilisées ensuite pour la détermination du degré de fusion d'un cycle courant.
Les Figures 11 et 12 illustrent deux techniques de fenêtrage différentes pour la comparaison du cycle à tester avec la référence en rythme sinusal.
La Figure 13 est un histogramme montrant la répartition des taux de fusion mesurés à chaque cycle cardiaque, évalués statistiquement sur une durée de plusieurs semaines.
La Figure 14 illustre, en superposition, une série d'histogrammes du même type que celui de la Figure 13, relevés pour des durées différentes de délai atrioventriculaire.
La Figure 15 est un organigramme donnant un exemple d'enchainement d'étapes du procédé d'ajustement dynamique du DAV selon l'invention.
La Figure 16 est un organigramme explicitant un contrôle complémentaire, mis en œuvre en cas de commutations multiples entre mode stimulé et mode spontané au niveau de l'oreillette.
La Figure 17 est un organigramme explicitant un autre contrôle complémentaire, mis en œuvre en cas d'instabilité détectée des intervalles PR ou AR.

On va maintenant décrire un exemple de mise en œuvre de la technique de l'invention.
En ce qui concerne ses aspects logiciels, l'invention peut être mise en œuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply CRT-P, Paradym CRT-D, Intensia CRT-D* et *Platinium CRT-D,* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en œuvre les fonctions de l'invention qui seront décrites ci-dessous. L'adaptation de ces appareils à la mise en œuvre des fonctions de l'invention est à la portée de l'homme du métier, et elle ne sera pas décrite en détail.

Le procédé de l'invention est mis en œuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

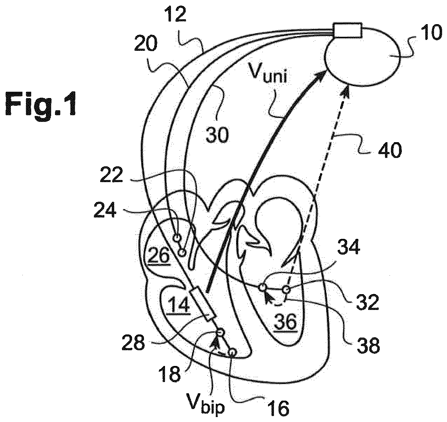

La Figure 1 illustre la configuration de stimulation à laquelle est appliquée l'invention, à savoir une stimulation biventriculaire, notamment afin de rétablir la synchronisation entre les deux ventricules.

Un générateur d'impulsions 10 est associé à une première sonde 12 implantée dans le ventricule droit 14. La tête de cette sonde comporte deux électrodes, à savoir une électrode distale (*tip*) 16 et une électrode proximale (*ring*) 18. Une deuxième sonde 20 est pourvue d'électrodes auriculaires de détection distale 22 et proximale 24 situées au niveau de l'oreillette droite 26 pour la détection des signaux dans cette cavité et l'application éventuelle d'une stimulation auriculaire (en variante, ces électrodes auriculaires 22, 24 peuvent être des électrodes flottantes disposées sur la sonde 12 au niveau de l'oreillette). Dans certaines configurations, la sonde ventriculaire droite 12 peut être également pourvue d'un bobinage (coin ventriculaire 28 formant électrode de défibrillation et permettant aussi de recueillir un signal endocavitaire (ce bobinage pouvant alors être utilisé à la place de l'électrode proximale *ring* 18).

Pour la stimulation du ventricule gauche, le générateur d'impulsions 10 est pourvu d'une troisième sonde 30, par exemple une sonde disposée dans le réseau coronaire, comportant une ou plusieurs électrodes 32, 34 disposées au voisinage du ventricule gauche 36 (dans le cas d'une sonde gauche "multi-électrodes", la sonde gauche peut également comporter une ou plusieurs électrodes intermédiaires située(s) dans une position médiane entre les électrodes 32 et 34). Il est ainsi possible d'assurer la stimulation concomitante, ou avec un léger décalage temporel contrôlé (délai interventriculaire DW), des deux ventricules droit et gauche pour rétablir la synchronisation entre ces deux cavités et améliorer l'hémodynamique générale du patient. Dans le cas d'une sonde gauche multi-électrodes, on peut également appliquer une stimulation multisite à gauche pour traiter un trouble du synchronisme intraventriculaire.

Il est également possible - et ce cas est celui considéré spécifiquement par la présente invention - de stimuler le seul ventricule gauche, lorsque la conduction atrioventriculaire droite native est préservée. Dans ce cas le ventricule droit se contracte spontanément, et la stimulation du ventricule gauche est contrôlée de manière que ce dernier se contracte en synchronisme avec le ventricule droit. Ceci réduit le besoin d'une stimulation droite, inutile voire délétère.

En ce qui concerne spécifiquement la stimulation du ventricule gauche, il est possible d'utiliser une configuration bipolaire (entre les deux électrodes 32 et 34 de la sonde 30) ou unipolaire (entre l'une des électrodes 32 ou 34 et le boitier can) du générateur 10, ou encore entre l'une des électrodes 32 ou 34 et une électrode de la sonde 12. Une sonde quadripolaire peut être également utilisée à ces mêmes fins. Des "vecteurs de stimulation" correspondants sont référencés 38 et 40 sur la Figure 1. Ces mêmes vecteurs peuvent être utilisés également pour le recueil d'un signal de dépolarisation ventriculaire gauche. On notera que dans le cas d'une sonde multipolaire il existe un grand nombre de vecteurs bipolaires et unipolaires possibles, définis à partir de chacune des électrodes (on peut également utiliser simultanément plusieurs vecteurs gauches, dans l'hypothèse évoquée plus haut d'une stimulation multisite à gauche). Comme cela sera décrit en détail plus bas, la technique de l'invention met en œuvre une combinaison de deux signaux d'électrogramme endocavitaire différents recueillis simultanément, en particulier des signaux issus de la même cavité ventriculaire, par exemple du ventricule droit.

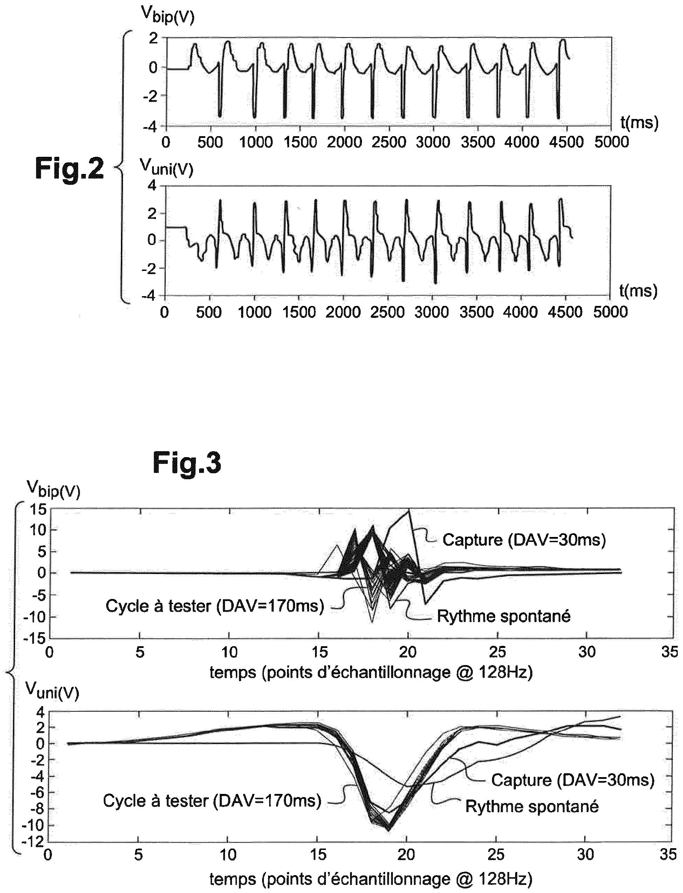

La Figure 2 illustre un exemple de tracés d'EGMs *Vbip* et *Vuni* observés respectivement sur la voie bipolaire ventriculaire et la voie unipolaire ventriculaire de la configuration de la Figure 1.

Les EGMs recueillis à cet effet dans le ventricule droit peuvent comprendre par exemple :

- une composante ventriculaire droite *Vbip,* dérivée d'un signal EGM *near-field* bipolaire recueilli entre l'électrode distale 16 et l'électrode proximale 18 de la sonde ventriculaire droite 12, et
- une autre composante ventriculaire droite *Vuni,* dérivée d'un signal EGM *far-field* unipolaire recueilli entre le bobinage de défibrillation 36 de la sonde ventriculaire droite 12 et le boitier métallique du générateur 10.

D'autres configurations peuvent être utilisées, à partir de signaux de type *far-field* (par exemple entre l'une des électrodes 16 ou 18 et le boitier 10, ou entre les électrodes 18 et 32) et de type *near-field* (par exemple entre deux électrodes 32 et 34 de la même sonde ventriculaire).

La Figure 3 illustre des exemples de signaux recueillis sur une voie bipolaire (*Vbip*) et sur une voie unipolaire (*Vuni*), pour différentes situations correspondant à une capture complète, à une perte totale de capture laissant apparaitre le rythme ventriculaire spontané, et pour un certain nombre de cycles intermédiaires à tester, avec présence d'une éventuelle fusion plus ou moins marquée, pour des DAV intermédiaires entre les deux situations de capture et de perte de capture.

On peut voir en particulier sur cette figure que l'information n'est pas la même sur les voies bipolaire et unipolaire et que, par exemple, les cycles en fusion (dans cet exemple, avec un DAV à 170 ms) sont beaucoup plus proches morphologiquement du rythme spontané que de la capture sur l'EGM bipolaire, et beaucoup plus proches de la capture sur l'EGM unipolaire.

En ne considérant que l'EGM unipolaire - comme dans la technique décrite par le EP 2 756 865 A1 précité -, la différence est beaucoup moins marquée entre cycles en fusion et cycles de référence avec capture complète ce qui conduirait, si l'on voulait quantifier la fusion, à un pourcentage de fusion (exprimé en pourcentage de capture) beaucoup plus important que la réalité.

Pour ces raisons notamment, il est avantageusement prévu dans cette invention d'utiliser une représentation 2D des deux EGMs.

La combinaison des deux composantes bipolaire et unipolaire en une caractéristique unique permet de disposer d'une référence contenant de façon plus globale toute l'information disponible issue de l'EGM, ce qui permet de quantifier de façon précise et robuste une éventuelle fusion présente avec le cycle cardiaque courant.

Plus précisément, les deux signaux bipolaire et unipolaire sont combinés en une caractéristique unique de type "boucle cardiaque" ou "vectogramme" (VGM), qui est la représentation dans un espace à deux dimensions de l'un des deux signaux EGM (en ordonnée) par rapport à l'autre (en abscisse). Chaque cycle cardiaque est alors représenté par un vectogramme dans le plan {*Vbip, Vuni*} ainsi défini, vectogramme dont la géométrie (forme de la courbe) fait donc abstraction de la dimension temporelle - qui n'intervient que comme un paramètre décrivant la manière dont la courbe est parcourue.

On soulignera que ce "vectogramme" (VGM), qui est obtenu à partir de signaux d'électrogramme (EGM) issu de sondes intracardiaques, ne doit pas être confondu avec le "vectocardiogramme" (VCG) qui est, lui, obtenu à partir de signaux d'électrocardiogramme (ECG) issu d'électrodes externes placées sur le thorax du patient.

La construction d'un VGM et son analyse pour quantifier des données cardiaques sont décrites par exemple dans Milpied et al., "Arrhythmia Discrimination in Implantable Cardioverter Defibrillators using Support Vector Machines applied to a new Représentation of Electrograms," IEEE Transactions on Biomédical Engineering, June 2011, 58(6):1797-1803. L'analyse d'un VGM a en outre été déjà proposée, comme exposé en introduction, par le EP 2 324 885 A1 (Sorin CRM) pour décider d'une suspicion de fusion afin d'invalider un test de capture.

On notera par ailleurs que l'analyse 'bidimensionnelle" ou "en deux dimensions" (2D) évoquée ici ne doit pas être entendue de manière en elle-même limitative. la technique décrite peut en effet s'appliquer aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

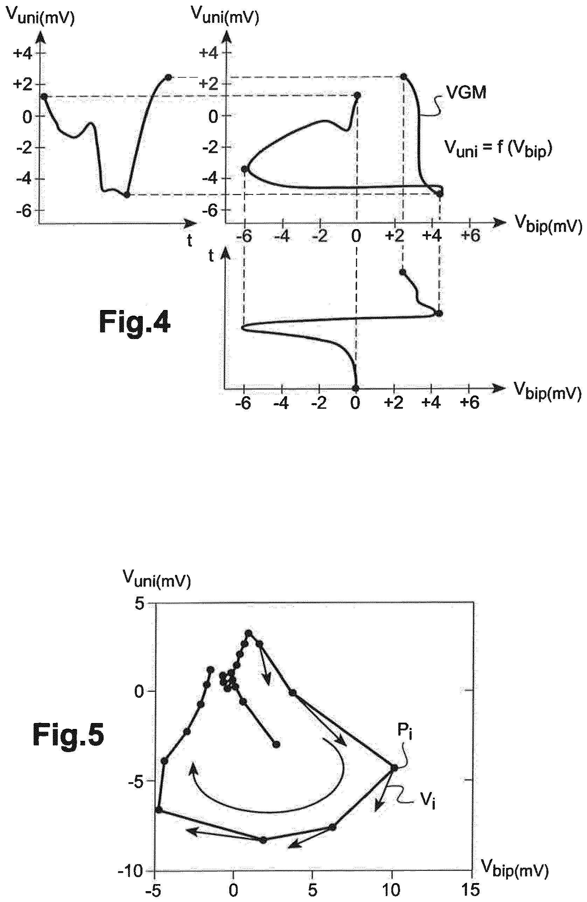

Comme illustré Figure 4, les signaux EGM $Vuni(t)$ et $Vbip(t)$ recueillis sont échantillonnés, et les échantillons successifs des deux composantes sont mémorisés puis combinés entre eux pour produire une courbe paramétrique (la caractéristique VGM) du type VGM = $(Vbip(t), Vuni(t))$ ou {x = $Vbip(t)$, y = $Vuni(t)$}.

Cette courbe est une courbe paramétrée par le temps, tracée à partir des variations de l'une des composantes temporelles (*Vuni*) en fonction de l'autre (*Vbip*). Elle constitue un vectogramme (VGM) représentatif du cycle cardiaque à analyser, et sera également désignée "caractéristique 2D paramétrique". Elle présente graphiquement la forme d'une boucle, le temps n'apparaissant plus que dans la manière dont la boucle est parcourue sur la durée du cycle.

En pratique, comme illustré Figure 5, l'échantillonnage produit un VGM en forme de polygone ouvert où chaque sommet correspond à un point d'échantillonnage de la mesure du signal *Vuni* et *Vbip* de l'EGM. Dans l'exemple de la Figure 5, l'échantillonnage est opéré à une fréquence de 128 Hz, ce qui donne environ 22 points de mesure pour un intervalle de temps de 164 ms, qui sont autant de valeurs qui peuvent être mises en mémoire pour être analysées (concrètement, il est possible de conserver en mémoire un plus grand nombre de points, par exemple 60 points, la comparaison entre deux vectogrammes étant en revanche opérée sur un nombre plus restreint de points, typiquement 11 ou 21 points).

On a également représenté sur la Figure 5 l'allure du vecteur vitesse $V_i$ en divers points successifs $P_i$ du VGM pour une fréquence d'échantillonnage de 128 Hz. En un point donné, la vitesse est une donnée vectorielle (la vitesse étant définie par son orientation et sa norme), et le vecteur vitesse peut être calculé en chaque point du VGM à partir d'un filtre discret qui approxime les dérivées premières *Vbip*(t)/*dt* et *Vuni*(t)/*dt* qui, pour une caractéristique échantillonnée, peuvent être calculées à partir du point précédent et du point suivant sur la courbe.

La caractéristique VGM recueillie est mémorisée sous forme d'une série de paramètres descripteurs basés sur les vecteurs vitesse en chaque point de la courbe et comprenant la norme du vecteur vitesse et l'orientation de ce vecteur vitesse, c'est-à-dire l'angle qu'il fait par rapport à l'axe des abscisses du VGM.

Pour quantifier la fusion, on procède avantageusement par une comparaison morphologique entre, d'une part, le VGM courant (mémorisé sous forme des valeurs des normes et des angles des vecteurs vitesse aux différents points d'échantillonnage) et, d'autre part, deux VGMs de référence (mémorisés sous forme de descripteurs homologues), l'un obtenu en situation de capture complète du ventricule et l'autre obtenu en situation de rythme exclusivement spontané dans le ventricule, c'est-à-dire avec un taux de fusion de 0 % en termes de pourcentage de capture.

La comparaison entre le VGM du cycle courant et ces deux VGMs de référence permet, en cas de détection d'une fusion (c'est-à-dire d'une situation qui n'est ni celle d'une capture complète ni celle d'un rythme spontané ventriculaire), d'évaluer un taux de fusion, permettant ainsi de quantifier par une métrique le décalage temporel plus ou moins important entre l'onde de dépolarisation évoquée consécutive à la stimulation, et l'onde de dépolarisation spontanée liée au rythme naturel.

La comparaison entre le VGM du cycle courant et l'un ou l'autre des VGMs de référence consistera à quantifier leurs ressemblances à partir :

- du coefficient de corrélation C entre les normes des vecteurs vitesse respectifs du VGM courant et du VGM de référence, et
- de la valeur moyenne θ de l'angle que font entre eux les vecteurs vitesse respectifs du VGM courant et du VGM de référence.

On pourra considérer que les courbes se ressemblent si C (qui reflète la corrélation entre les normes des vecteurs vitesse est suffisamment grand, c'est-à-dire proche de l'unité, et θ (qui reflète les écarts angulaires d'orientation) est suffisamment petit, c'est-à-dire proche de zéro. Plus la valeur de C s'éloignera de 1 et plus celle de θ s'éloignera de 0, plus les deux VGMs seront dissemblables.

On notera que l'utilisation des paramètres C et θ pour opérer la comparaison entre deux VGMs n'est pas limitative, et que d'autres paramètres peuvent être utilisés. De la même façon, l'utilisation combinée de C et θ est particulièrement avantageuse, mais il serait également possible d'évaluer la ressemblance des VGMs à partir d'un seul des deux paramètres C ou θ, ou d'un nombre plus important de paramètres, trois paramètres et plus. Les descripteurs C et θ sont cependant préférés, en raison de leur faible sensibilité aux artefacts et de la relative facilité qu'il y a à les calculer.

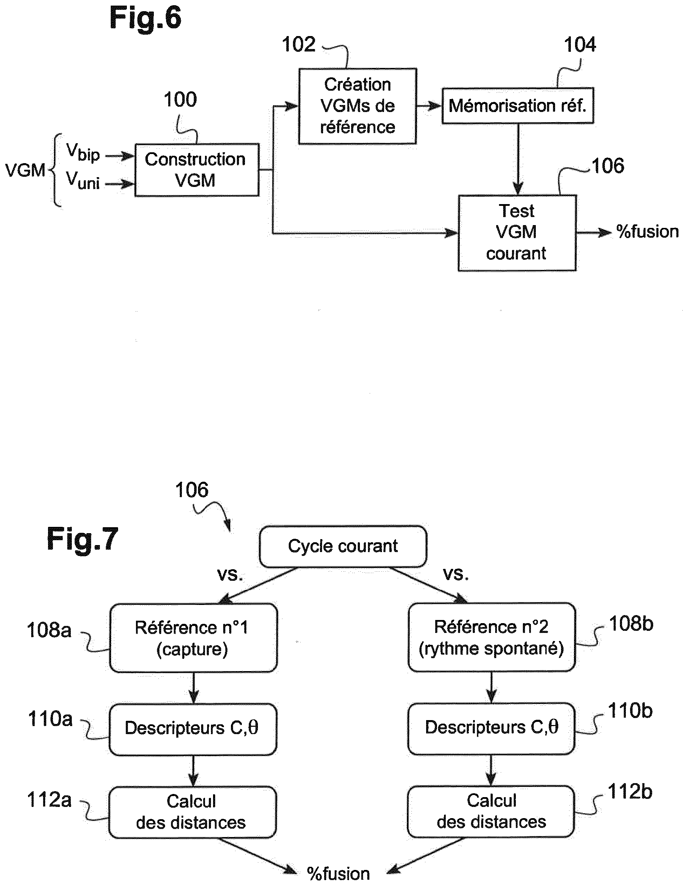

La Figure 6 illustre de façon très générale la manière dont est mise en œuvre cette technique préférentielle de quantification.

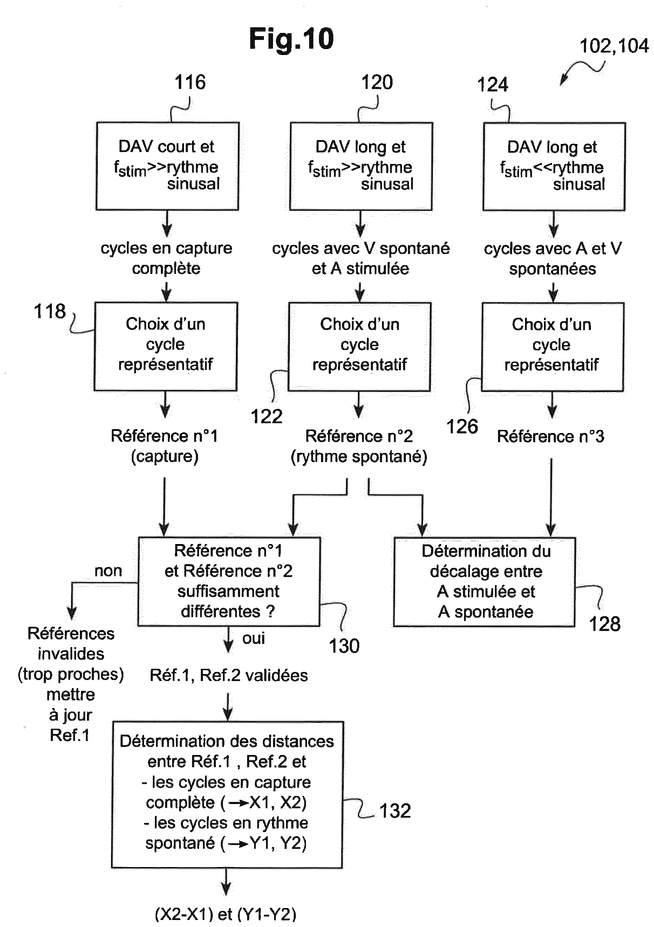

À partir des EGMs bipolaire et unipolaire recueillis, un VGM est construit (bloc 100). Au préalable, deux VGMs de référence sont constitués dans des conditions stables (rythme sinusal lent, de préférence la nuit) (bloc 102, détaillé Figure 10) et conservés en mémoire (bloc 104). Les VGMs courants seront ultérieurement comparés (bloc 106) aux deux VGMs de référence mémorisés, pour produire en sortie une métrique quantifiant un taux de fusion compris entre 0 et 100 %.

Le "taux de fusion" peut être exprimé uniquement en termes de taux de rythme spontané - c'est-à-dire qu'un taux de fusion de 0 % correspondra à une capture complète et un taux de fusion de 100 % correspondra à une situation de rythme exclusivement spontané. On peut également exprimer ce taux de fusion par rapport à une situation de capture, le taux de fusion variant alors de 100 % à 0 % dans le sens inverse.

La Figure 7 illustre le test du bloc 106, sous forme d'un organigramme simplifié présentant la séquence d'étapes exécutées pour l'analyse d'un cycle courant.

Le VGM du cycle courant est comparé à la fois au VGM de référence correspondant à une situation de capture complète, ci-après "Référence n°1" (bloc 108a) et au VGM correspondant à une situation de rythme spontané ventriculaire, ci-après "Référence n°2" (bloc 108b).

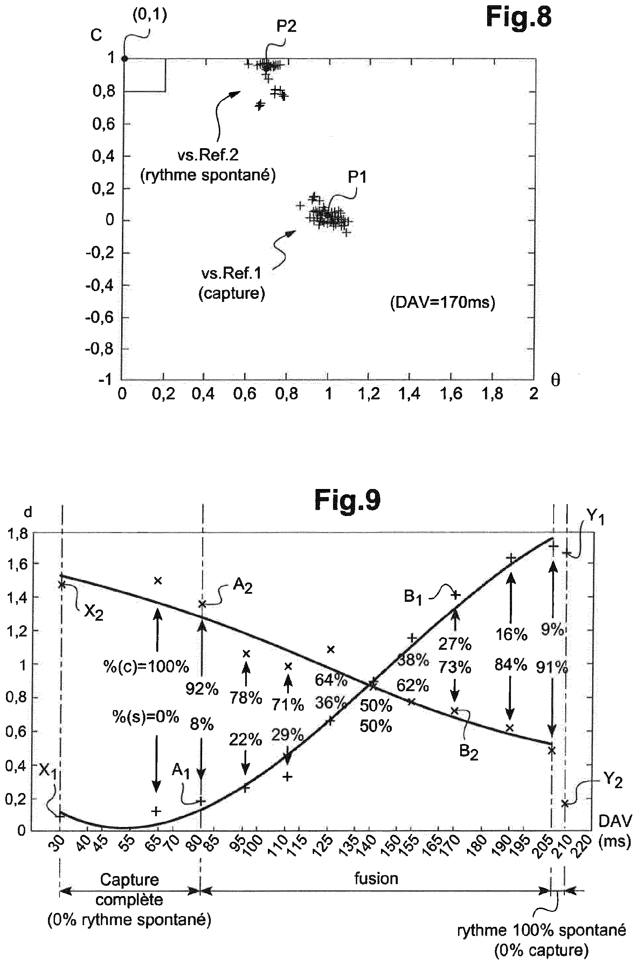

Ces deux comparaisons produisent deux couples respectifs de descripteurs C et θ (blocs 110a et 110b), qui peuvent être représentés dans un espace bidimensionnel illustré Figure 8. L'étape suivante (blocs 112a et 112b) consiste à calculer les distances des divers points [C,θ] de l'espace bidimensionnel correspondant aux descripteurs obtenus par rapport au point d'origine [0,1] de ce même espace, afin de déterminer, à partir de ces distances et de la manière que l'on décrira plus bas, une métrique quantifiant un taux de fusion du cycle courant.

La Figure 8 illustre, dans l'espace bidimensionnel dont les deux dimensions correspondent aux deux descripteurs C et θ, un exemple de répartition des points obtenus pour quelques dizaines de cycles courants avec un même DAV, ajusté en l'espèce à 170 ms. Cet exemple montre deux nuages de points, l'un obtenu par comparaison des cycles courants avec la Référence n°1 (capture complète), l'autre par comparaison avec la Référence n°2 (rythme spontané ventriculaire). Les points P1 et P2 indiquent les barycentres respectifs de ces deux nuages de points.

Le point [0,1] correspond à une situation théorique idéale où le cycle courant serait identique à l'un ou l'autre des cycles de référence, avec un coefficient de corrélation C égal à l'unité et un angle moyen θ nul entre les vecteurs vitesse respectifs des deux VGMs.

La comparaison des VGMs des cycles courants avec chacune des Références 1 et 2 est évaluée :

- soit par comparaison avec des seuils prédéterminés sur C et/ou $\theta$ : on considère par exemple que les courbes se ressemblent si C > Seuil 1 et $\theta$ < Seuil 2 ;
- soit par une relation entre C et $\theta$ : on considère par exemple que les courbes se ressemblent si C > $\theta$ ;
- soit, avantageusement, à l'aide de la distance calculée dans le plan [C, $\theta$] des descripteurs entre le point courant et le point [0,1] : plus cette distance est petite, plus les VGMs se ressemblent.

Ainsi, si dans l'exemple illustré on considère la distance entre chacun des points P1 et P2 et le point d'origine [0,1], on constate que les résultats des deux comparaisons respectives montrent que le nuage de points de la comparaison avec la Référence n°2 (rythme spontané) est plus proche de cette origine que le nuage de points de la comparaison avec la Référence n°1 (capture complète), ce qui signifie que les cycles courants sont, en fusion, plus proches d'un rythme ventriculaire spontané que d'une capture complète - donc avec un taux de fusion, exprimé en pourcentage de capture, inférieur à 50 % - sans être toutefois suffisamment proche du point [0,1] pour conclure à un rythme purement spontané.

La Figure 9 est une représentation dans laquelle on a fait varier le DAV entre toutes les valeurs d'ajustement possibles, depuis une valeur minimale DAV = 30 ms produisant en toutes circonstances une capture complète du ventricule, jusqu'à une valeur maximale de DAV suffisamment longue pour laisser s'exprimer le rythme spontané. En ordonnée est portée la distance *d* entre l'origine [0,1] de l'espace bidimensionnel de la Figure 8 et le point [C,$\theta$] résultant de la comparaison avec la Référence n°1 (points représentés par des "+") ou avec la Référence n°2 (points représentés par des "x").

Pour un DAV minimal DAV = 30 ms en capture ventriculaire complète, on obtient respectivement les repères X1 et X2, et pour un DAV maximal (ici de 210 ms) on obtient les repères respectifs Y1 et Y2.

Sur les figures ont également été indiqués les pourcentages de fusion, exprimés en termes de pourcentages de capture ("%(C)") et en termes de pourcentages de rythme spontané ("%(S)").

Si le cycle courant en fusion est plus proche d'une capture que d'un rythme spontané (partie gauche de la Figure 9, cas des repères A1 et A2), alors le pourcentage de capture est supérieur à 50 % et le pourcentage de rythme spontané est inférieur à 50 %. Inversement, si le cycle courant en fusion est plus proche d'un rythme spontané que d'une capture (partie droite de la Figure 10, cas des repères B1 et B2 correspondant aux points P1 et P2 de la Figure 9), alors le pourcentage de capture est inférieur à 50 % et le pourcentage de rythme spontané supérieur à 50 %.

Pour quantifier le degré de fusion, on va considérer l'écart entre les repères X1 et X2 obtenus en capture complète, écart qui est dans cet exemple de l'ordre de X2-X1 = 1,4. De la même façon, l'écart entre les repères Y1 et Y2 en rythme spontané complet est de l'ordre de Y1-Y2 = 1,6. Dans le cas d'un cycle courant en fusion plus proche de la capture que du rythme spontané (repères A1 et A2, où A2 > A1), on devrait toujours avoir A2-A1 < 1,4, et l'on devrait s'éloigner de la capture (A1 > X1) et se rapprocher du rythme spontané (A2 < X2).

Si A2 > A1 et A2-A1 < X2-X1 et si A1 > X1 et A2 < X2 (à une tolérance près, par exemple une tolérance de 0,1 près), alors le pourcentage de capture sera :

$$50 + [50*(A2-A1)/(X2-X1)]$$

Dans le cas contraire, aucun degré de fusion ne sera calculé pour le cycle en question, qui correspond peut-être à une extrasystole et ne suit pas la progression attendue. Un compteur pourra être incrémenté, car si ce cas de figure est trop fréquent, il peut signifier que les références et distances de référence X2 et X1 sont à remettre à jour.

On pourra considérer qu'un pourcentage de capture supérieur à un seuil donné, par exemple supérieur à 90 %, correspond à une situation de capture complète, et non de fusion.

Le pourcentage de rythme spontané peut alors être calculé comme étant :

100 - le pourcentage de capture.

Si le cycle courant en fusion est plus proche du rythme spontané que de la capture (repères B1 et B2, où B1 > B2), pour calculer le pourcentage de rythme spontané on peut se baser sur la distance entre les repères en rythme spontané Y1 et Y2.

Pour un cycle courant en fusion, on devrait toujours obtenir une valeur B1-B2 < 1,6 et l'on devrait se rapprocher de la capture (B1 < Y1) et s'éloigner du rythme spontané (B2 > Y2).

Si B1 > B2 et B1-B2 < Y1-Y2 et si B1 < Y1 et B2 > Y2 (à une tolérance près, par exemple une tolérance de 0,1 près), alors le pourcentage de rythme spontané sera :

$$50 + [50 * (B1-B2)/(Y1-Y2)].$$

Dans le cas contraire, aucun degré de fusion ne sera calculé pour le cycle en question. Un compteur pourra être incrémenté, car si ce cas de figure est trop fréquent, il peut signifier que les références et distances de référence Y2 et Y1 sont à remettre à jour.

On pourra considérer qu'un pourcentage de rythme spontané supérieur à un seuil donné, par exemple supérieur à 95 %, correspond à une situation de rythme spontané et non de fusion.

Le pourcentage de capture peut alors être calculé comme étant :

100 - le pourcentage de rythme spontané.

Le taux de fusion final pourra être exprimé soit en termes de taux de capture, soit en termes de taux de rythme spontané, soit par une combinaison de ces deux taux.

En référence aux Figures 10 à 12, on va maintenant décrire la manière d'obtenir les deux références utilisées pour la détermination du taux de fusion.

Ces références sont créées et actualisées de façon périodique et en conditions stables (en particulier la nuit), par exemple une fois par jour ou une fois par semaine.

Pour établir la référence en capture complète (Référence n°1), le DAV est programmé à la valeur la plus courte possible, par exemple DAV = 30 ms, de préférence avec stimulation de l'oreillette à une fréquence de stimulation notablement supérieure au rythme sinusal (bloc 116) de manière à s'éloigner le plus possible des conditions d'apparition d'une fusion éventuelle.

On enregistre dans ces conditions une pluralité de cycles en capture complète, qui sont ensuite comparés de manière à établir ou choisir un cycle représentatif unique (bloc 118). Une première méthode consiste à enregistrer plusieurs VGMs et à les comparer deux à deux, puis vérifier qu'un nombre minimum de VGMs, par exemple deux VGMs se ressemblent. Une autre méthode consiste à rechercher, au fur et à mesure de l'enregistrement des cycles, deux VGMs qui se ressemblent, avec un nombre maximum de VGMs à tester, par exemple cinq VGMs ; si l'on constate une ressemblance suffisante entre les cycles, alors la Référence n°1 est créée et mémorisée, soit en sélectionnant l'un des cycles, soit en moyennant les cycles qui se ressemblent.

Pour la référence en rythme spontané (Référence n°2), un DAV très long est programmé, par exemple DAV = 300 ms, de manière à laisser s'exprimer le rythme spontané avant toute stimulation (bloc 120). Une pluralité de cycles sont ainsi produits de la même façon que pour les cycles en capture complète, et la Référence n°2 est créée pareillement par détermination d'un cycle représentatif unique (bloc 122) après comparaison des différents cycles produits.

Toutefois, pour l'établissement de cette Référence n°2 une fenêtre de mesure plus large est nécessaire, car la partie de l'EGM utilisée pour la comparaison ultérieure des cycles à tester dépendra du DAV desdits cycles à tester. De plus, l'instant de survenue de la dépolarisation n'est pas le même dans le cas d'un événement auriculaire stimulé (évènement A) que dans celui d'une dépolarisation spontanée de l'oreillette (évènement P).

Une première possibilité consiste à établir deux références distinctes en rythme ventriculaire spontané, l'une avec stimulation de l'oreillette et l'autre avec dépolarisation spontanée de l'oreillette (rythme sinusal).

Une autre solution, préférée, consiste à établir une référence unique, par exemple avec dépolarisation stimulée de l'oreillette, et à prévoir une valeur de compensation temporelle ou *offset*, déterminée comme étant la différence des temps de conduction dans le ventricule constatés pour une dépolarisation spontanée de l'oreillette (P) et pour une dépolarisation stimulée de l'oreillette (A) : *offset* = AR-PR.

Ce décalage est établi par une programmation temporaire d'un DAV long avec une fréquence de stimulation très inférieure au rythme sinusal (bloc 124), puis sélection, comme précédemment, d'un cycle représentatif (bloc 126) pour obtenir une référence provisoire (Référence n°3) permettant de déterminer le décalage ci-dessus (bloc 128).

Dans une variante simplifiée, ce décalage peut être établi lors du calcul de la Référence n° 2 en mesurant des intervalles AR, ainsi que des intervalles PR, sans Référence n°3 mais seulement dans les conditions de DAV long et faible fréquence de stimulation (bloc 124), ceci sur quelques cycles (8 cycles par exemple) et en prenant la différence entre la valeur moyenne des intervalles AR et la valeur moyenne des intervalles PR. Une fois établies les deux références Référence n°1 et Référence n°2, une dernière étape consiste à comparer ces deux références en appliquant un DAV fictif de 30 ms et un *offset* si nécessaire (de manière que la condition de l'oreillette et le DAV soient les mêmes). Les deux références sont alors comparées (bloc 130) :

- si la Référence n°1 est bien en capture complète, alors la dépolarisation de la Référence n°2 doit arriver plus tard que celle de la Référence n°1 (pas de superposition dans le temps), la comparaison des courbes devant donc donner une différence importante entre les références. Les références sont alors validées et analysées (bloc 132) de manière à déterminer les repères X1, X2 et Y1, Y2 et les écarts X2-X1 et Y1-Y2 (bloc 132) ;

- si, en revanche, la distance dans l'espace bidimensionnel des descripteurs C et $\theta$ entre le point courant et le point [0,1] est inférieure à un seuil donné, par exemple inférieure à l'unité, alors on considère que les références sont trop proches, la Référence n°1 étant sans doute déjà en fusion et non pas en capture complète. Il ne sera donc pas possible de déterminer un degré de fusion correct et la Référence n°1 est alors invalidée comme référence de capture complète.

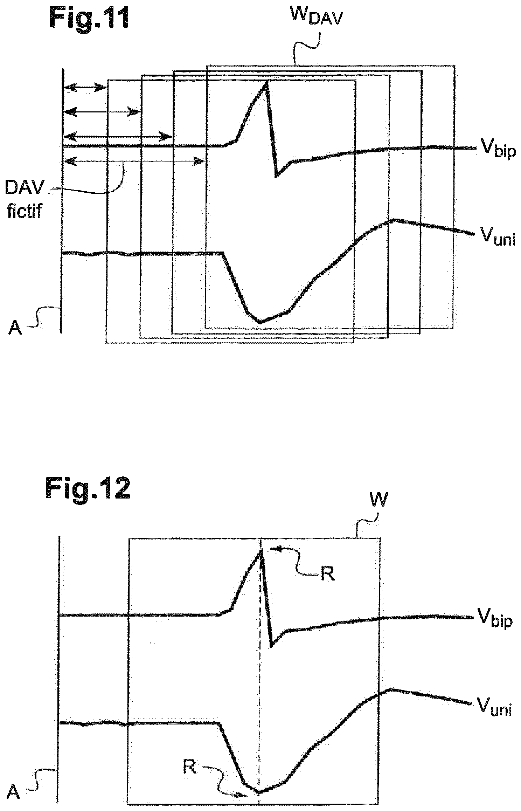

Dans ce dernier cas, il est possible de réitérer le calcul de la Référence n°1 en produisant une pluralité de cycles supplémentaires en mode VVI à une fréquence supérieure à la fréquence de base du patient, par exemple à une fréquence de 100 bpm permettant de récupérer une référence en capture complète. Il suffira alors de comparer à la Référence n°2 tous les cycles ainsi obtenus et de prendre celui qui en diffère le plus tout en étant très proche de la réponse évoquée avec un DAV = 30 ms, cette sélection étant opérée comme précédemment par application de seuils aux valeurs de C et θ. Une autre possibilité consiste à utiliser une Référence n°1 plus ancienne (si elle existe) et la revalider avec la nouvelle Référence n°2. Les Figures 11 et 12 illustrent deux techniques de fenêtrage différentes pour la comparaison du cycle à tester avec la référence en rythme spontané.

En effet, lorsqu'il s'agit de comparer un cycle courant stimulé avec la référence en rythme spontané, un DAV est appliqué au cycle courant alors que les cycles de la référence avaient été obtenus sans DAV.

Comme illustré Figure 11, pour comparer des cycles à tester stimulés avec des DAV variables à la Référence n°2 en rythme spontané, le dispositif applique un "DAV fictif (fictif, car on est en rythme spontané) ajouté à l'*offset* en cas de non-stimulation de l'oreillette, produisant ainsi un décalage de la fenêtre $W_{DAV}$ d'analyse des EGMs bipolaire et unipolaire. On peut ainsi disposer d'une Référence n°2 unique quelle que soit la valeur du DAV programmable (de 30 à 250 ms typiquement). Par exemple, si l'on veut tester un cycle avec stimulation de l'oreillette et un DAV à 125 ms, on utilisera la Référence n°2 avec un DAV fictif de 125 ms. En effet, le séquencement des EGMs par rapport au marqueur auriculaire A est aussi important que la morphologie des signaux lorsqu'il s'agit de comparer entre eux deux VGMs obtenus à partir de signaux EGM bipolaire et unipolaire respectifs.

La Figure 12 illustre une variante consistant à utiliser, au lieu de la fenêtre mobile de la Figure 11, une fenêtre unique W centrée sur le pic caractéristique (extremum R) du signal EGM *Vbip* ou *Vuni,* indépendamment du type de dépolarisation auriculaire, spontanée ou stimulée. Toutefois, cette solution n'est pas optimale dans la mesure où elle ne tient pas compte du délai de conduction.

*Application de la mesure du taux de fusion au contrôle du DAV dans un stimulateur CRT à stimulation du ventricule gauche*

On va maintenant décrire un exemple spécifique d'exploitation des données de taux de fusion obtenues dynamiquement à chaque cycle cardiaque de la manière qui a été décrite plus haut.

L'application que l'on va exposer n'est toutefois pas limitée à cette technique particulière de détermination du taux de fusion, et elle peut être mise en œuvre sur la base de mesures du taux de fusion obtenues d'une autre manière.

Plus précisément, l'application objet de la présente invention se situe dans le contexte d'une thérapie CRT avec stimulation d'une cavité pour se synchroniser sur le rythme spontané de la cavité opposée.

Dans l'exemple qui suit, on considérera une stimulation du seul ventricule gauche synchronisé sur la dépolarisation spontanée du ventricule droit, mais ce cas n'est pas limitatif, l'invention pouvant s'appliquer aussi bien, comme indiqué en introduction, par exemple au cas de la resynchronisation des deux oreillettes entre elles, ou à celui d'une stimulation du ventricule droit synchronisée sur la dépolarisation spontanée du ventricule gauche.

Cette thérapie peut être mise en œuvre lorsque la conduction atrioventriculaire droite native est préservée : le ventricule droit se contracte alors spontanément, et la stimulation du ventricule gauche est déclenchée et contrôlée de manière que ce dernier se contracte en synchronisme avec le ventricule droit.

On notera que cette technique peut être mise en œuvre aussi bien à partir d'une contraction spontanée de l'oreillette, le rythme étant alors le rythme sinusal naturel spontané, que d'une contraction de l'oreillette déclenchée par une impulsion de stimulation générée par le dispositif implanté.

En tout état de cause, la conduction atrioventriculaire droite étant par hypothèse préservée, dès lors qu'a été obtenue une contraction de l'oreillette, stimulée (évènement A) ou spontanée (évènement P), le ventricule droit se contractera spontanément (évènement R) après un intervalle PR ou AR suivant la contraction de l'oreillette droite.

Dans une telle situation, il convient de chercher à favoriser la fusion de la stimulation du ventricule gauche avec la conduction spontanée du ventricule droit.

Le délai atrioventriculaire DAV, qui commande l'instant de la stimulation du ventricule gauche, devra donc être adapté pour garantir la fusion et obtenir l'effet mécanique souhaité de resynchronisation des deux ventricules.

Toutefois, en pratique le degré de fusion peut varier d'un cycle cardiaque à l'autre en fonction du mode de fonctionnement programmé pour le dispositif, de l'activité du patient, etc.

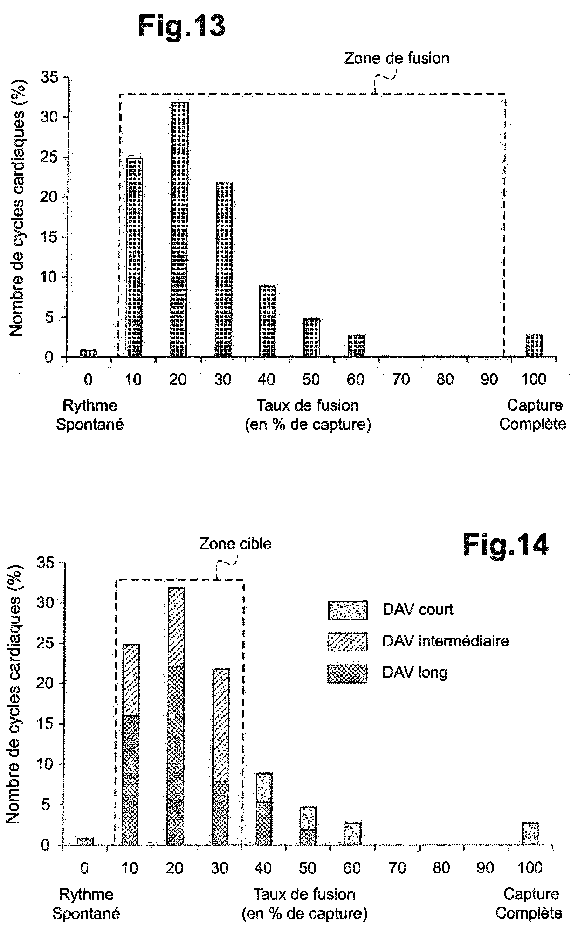

La Figure 13 est un histogramme typique de valeurs du taux de fusion (exprimé en pourcentage de capture) relevées sur une période longue, de l'ordre de trois mois. La "zone de fusion" correspond à la zone dans laquelle on considère qu'il y a une fusion, plus ou moins importante, les classes extrêmes 0 et 100, correspondant respectivement à un rythme spontané pur et à une capture complète, étant considérées comme une absence de fusion.

Dans le cas d'une stimulation CRT du ventricule gauche, on va chercher à restreindre cette zone de fusion à une "zone cible" correspondant à un nombre réduit de classes de l'histogramme, par exemple un degré de fusion compris entre 10 % et 30 % (exprimé en pourcentage de capture). Cette zone cible, correspondant à un degré optimal de fusion recherché, variera en fonction de l'application, et sera déterminée *a priori* à partir de mesures hémodynamiques (échographie, mesure de $dP/dt_{max}$, etc.), de mesures électriques (électrocardiogramme, mesure des intervalles PR, du temps de conduction LVp-RVs) ou de mesures délivrées par d'autres capteurs, ou de valeurs paramétrées manuellement par le médecin. La valeur cible, correspondant au degré optimal de fusion, pourra être affectée d'une certaine tolérance pour tenir compte des variations de mesure et de précision cycle à cycle : par exemple, pour une cible de taux de fusion de 20 % (en pourcentage de capture) on peut prévoir une tolérance de 20 ± 10 %, soit une zone cible comprise entre 10 et 30 % de taux de fusion (en pourcentage de capture).

La Figure 14 illustre un histogramme du même type que celui de la Figure 13, où on a délimité une zone cible correspondant à cet exemple, à savoir 10 à 30 % de taux de fusion (en pourcentage de capture).

En pratique, si l'on fait varier le DAV, on constate que cet histogramme se modifie :

- pour un DAV court, un nombre important de cycles cardiaques se situent en dehors de la zone cible ;
- pour un DAV de valeur intermédiaire, la quasi-totalité des cycles cardiaques sont situés dans la zone cible ;
- pour un DAV long, on retrouve, comme dans le cas du DAV court, un nombre non négligeable de cycles situés en dehors de la zone cible.

Le but de la technique que l'on va exposer consiste, sur la base d'une mesure dynamique du taux de fusion, opérée cycle à cycle, à ajuster le DAV de façon automatique pour maximiser le nombre de cycles cardiaques présentant un taux de fusion compris dans la zone cible. Il est vraisemblable que le degré de fusion varie peu lorsque les conditions adrénergiques du patient sont stables, et pour préserver la batterie du dispositif, il est avantageux de ne mesurer le taux de fusion que tous les C cycles ($1 \leq C \leq 60$ par exemple). La fréquence (C) d'évaluation du taux de fusion peut être réduite (C peut passer de 8 à 1 par exemple) sur détection d'un effort et accélération du rythme cardiaque du patient, ou lorsque le taux de fusion entre deux mesures est très variable.

La décision de modifier le DAV pour maintenir le degré de fusion autour de la valeur cible pourra être prise cycle à cycle, ou bien selon un critère majoritaire de X cycles sur Y (par exemple 6 cycles sur 8). Essentiellement, après avoir mesuré le degré de fusion du cycle courant, le dispositif évalue l'écart entre la valeur mesurée et la valeur cible. Si cet écart se situe dans le sens de la capture (vers la droite sur l'histogramme de la Figure 14), il conviendra d'augmenter le DAV pour le cycle suivant ; inversement, pour une variation dans le sens du rythme spontané (vers la gauche sur l'histogramme de la Figure 14) il conviendra de réduire le DAV. L'incrément de variation du DAV peut être fixe (1 ou 2 pas prédéterminés), ou variable en fonction de l'écart entre le taux de fusion courant mesuré et le taux de fusion cible.

Si le DAV est modifié cycle à cycle en fonction du degré de fusion du cycle précédent, le dispositif s'adapte alors automatiquement et rapidement au rythme cardiaque et à l'état adrénergique du patient.

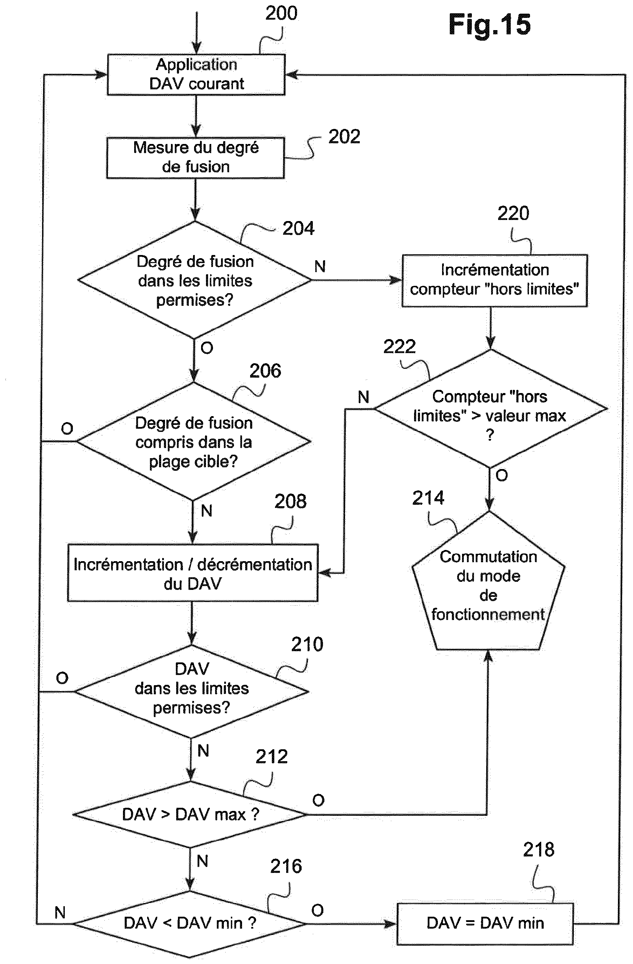

La Figure 15 est un exemple d'organigramme mettant en œuvre cette manière de procéder.

L'étape 200 correspond à l'application d'une valeur de DAV courant à un cycle cardiaque. Le degré de fusion de ce cycle est mesuré (bloc 202), puis il est comparé à des bornes limites prédéterminées, par exemple 95 % (en rythme spontané) en limite supérieure et 30 % (en rythme spontané) en limite inférieure.

Si cette condition est vérifiée, alors on examine si le degré de fusion mesuré est ou non compris dans la plage cible (bloc 206), à savoir entre 10 et 30 % de capture dans l'exemple exposé plus haut. Dans l'affirmative, aucune action particulière n'est prise (retour au bloc 200). Dans la négative, il convient de modifier le DAV pour ramener le degré de fusion dans la plage cible.

Le DAV est alors, selon le cas, incrémenté ou décrémenté (bloc 208) comme expliqué plus haut.

Avant d'appliquer la valeur modifiée du DAV, on vérifie que celui-ci reste dans des limites permises maximale et minimale $DAV_{max}$ et $DAV_{min}$ (bloc 210).

Si la borne supérieure DAVmax est atteinte (bloc 212), ceci résulte très vraisemblablement de l'apparition d'un bloc atrioventriculaire, imposant de modifier le mode de fonctionnement du dispositif (bloc 214) par exemple pour déclencher une stimulation forcée du ventricule droit. On notera que cette technique permet, par une surveillance en continu du degré de fusion, d'identifier la survenue de blocs atrioventriculaires sans qu'il soit nécessaire de monitorer l'intervalle PR ou AR, évitant ainsi un risque de stimulation insuffisante du patient.

Si la borne inférieure $DAV_{min}$ est atteinte (bloc 216), le DAV n'est pas réduit, mais limité à cette valeur minimale (bloc 218). On notera que cette limite $DAV_{min}$ peut être une limite variable, notamment en fonction de l'activité du patient, par exemple $DAV_{min}$ = 65 ms au repos et $DAV_{min}$ = 32 ms à l'exercice.

De plus si, comme détecté au bloc 204, le degré de fusion d'une majorité de cycles sur par exemple la dernière heure (ou demi-heure, ou tout autre intervalle de temps) tombe en dehors de bornes limites, un compteur est incrémenté (bloc 220) et périodiquement testé (bloc 202). S'il est avéré qu'un nombre important de cycles se situent hors de la zone cible, le mode de stimulation n'est vraisemblablement pas approprié, et un autre mode de fonctionnement est choisi (bloc 214), par exemple une capture complète biventriculaire.

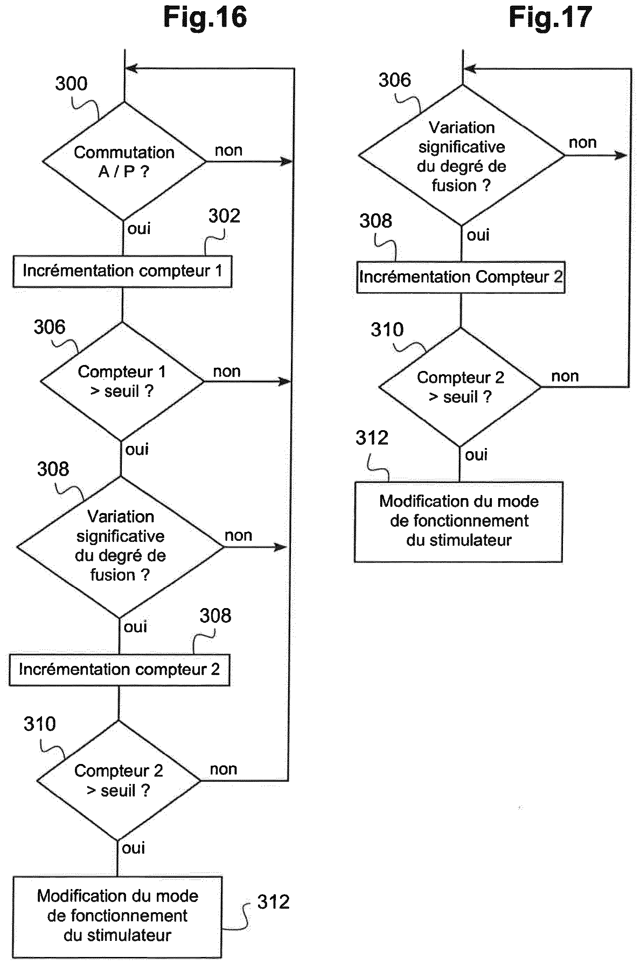

La Figure 16 est un organigramme explicitant un contrôle complémentaire, mis en œuvre en cas de commutations multiples entre mode stimulé et mode spontané au niveau de l'oreillette.

Comme on l'a exposé plus haut en référence notamment aux Figures 11 et 12, en cas de passage d'un rythme auriculaire spontané (évènements P) à un cycle avec stimulation de l'oreillette (évènement A), ou *vice versa,* le dispositif applique au DAV courant un *offset* ou décalage, préalablement déterminé, pendant la création des références (comme décrit à propos de la Figure 10), afin de garder un degré de fusion comparable entre les cycles en rythme spontané et en rythme stimulé. Cependant, si la fréquence de stimulation auriculaire est proche de la fréquence sinusale du patient, alors il peut arriver que le dispositif commute à plusieurs reprises entre mode avec stimulation de l'oreillette et mode avec contraction spontanée de l'oreillette. On peut alors considérer que pour la plupart des cycles on obtient une fusion entre le rythme sinusal et la stimulation auriculaire, et donc que l'*offset* n'est pas adéquat et produit un DAV trop long qui privilégie la dépolarisation spontanée du ventricule.

Ainsi, comme illustré Figure 16, à chaque commutation A/P détectée (bloc 300), on incrémente un premier compteur (bloc 302) et si la valeur de ce compteur dépasse un seuil prédéterminé (bloc 304), par exemple dix commutations dans la dernière minute, on évalue la variation du degré de fusion (bloc 306) entre cycles consécutifs :

- si cette variation est inférieure à une tolérance donnée, par exemple 10 %, alors on considère que la variation n'est pas significative ;
- dans le cas contraire, à chaque fois que cette variation change de signe un compteur est incrémenté (bloc 308).

Si le compteur dépasse un seuil donné (bloc 310), par exemple dix variations significatives dans la dernière minute, alors on considère que le degré de fusion est trop instable. Une action est alors entreprise (bloc 312), par exemple :

- ne plus appliquer l'*offset* lors des changements suivants de rythme auriculaire spontané vers un rythme auriculaire stimulé ;
- ou bien reprogrammer automatiquement la fréquence de stimulation auriculaire : selon le cas en abaissant cette fréquence afin de privilégier le rythme spontané du patient, ou en accélérant cette fréquence afin d'éviter des commutations inutiles du fait d'une trop grande proximité avec la fréquence sinusale.

La Figure 17 est un organigramme explicitant un autre contrôle complémentaire, mis en œuvre en cas d'instabilité détectée des intervalles PR ou AR.

On a décrit à la Figure 10 la manière d'élaborer les deux références, à savoir la Référence n° 1 en capture et la Référence n° 2 en rythme spontané. Lorsque la Référence n° 2 est élaborée avec un DAV fictif (comme illustré Figure 11), les références obtenues correspondent à un intervalle PR précis. Mais si l'intervalle PR est instable, pour des mêmes paramètres de stimulation, le degré de fusion ne sera pas le même d'un cycle à l'autre.

Il peut être de ce fait intéressant de mettre en évidence l'instabilité de l'intervalle PR ou AR, sans modification des paramètres courants de stimulation, ni inhibition de la stimulation.

Comme dans le cas de la Figure 16, on peut monitorer les variations de pourcentage de capture entre cycles consécutifs (étapes 306 et 310 de la Figure 17, identiques aux mêmes étapes décrites plus haut à propos de la Figure 16).

Lorsque le degré de fusion devient trop instable (bloc 310), alors il convient (bloc 312) :

- soit de commuter vers un autre mode de stimulation, par exemple en capture complète biventriculaire ;
- soit de rester en stimulation du seul ventricule gauche tant que le degré de fusion reste dans les limites autorisées. Dans ce dernier cas, il pourra être préférable d'utiliser une unique référence en rythme spontané (Référence n° 2), centrée sur un point caractéristique du signal.

## Revendications

1. Un dispositif médical implantable actif de type resynchroniseur cardiaque, comprenant :

    - des moyens de stimulation ventriculaire (10, 12, 30), aptes à produire des impulsions de stimulation destinées à être délivrées au moins au ventricule gauche (36) d'un patient porteur du dispositif ;
    - des moyens (10, 12, 20, 30) de détection d'événements auriculaires et ventriculaires ; et
    - des moyens d'application d'un délai atrioventriculaire, DAV, entre un événement auriculaire détecté ou stimulé et la délivrance d'une impulsion de stimulation ventriculaire,

    dispositif **caractérisé en ce qu'**il comprend en outre :

    - des moyens de quantification (202) d'un degré de fusion entre la délivrance d'une impulsion de stimulation à une cavité, gauche ou droite, et une contraction spontanée d'une autre cavité, opposée, respectivement droite ou gauche, ces moyens étant aptes à calculer une valeur de taux de fusion comprise entre deux valeurs extrêmes correspondant respectivement à une situation de capture complète et à une situation de dépolarisation spontanée de ladite cavité ; et
    - des moyens d'adaptation dynamique du DAV (206, 208), aptes à modifier la valeur du DAV appliqué à la délivrance de ladite impulsion de stimulation ventriculaire, en fonction d'une comparaison opérée entre i) la valeur courante du taux de fusion calculée par les moyens quantificateurs, et ii) une valeur cible de taux de fusion.

2. Le dispositif de la revendication 1, dans lequel les moyens de quantification (202) d'un degré de fusion sont des moyens de quantification (202) d'un degré de fusion entre la délivrance d'une impulsion de stimulation au ventricule gauche, et une contraction spontanée du ventricule droit, ces moyens étant aptes à calculer une valeur de taux de fusion comprise entre deux valeurs extrêmes correspondant respectivement à une situation de capture complète et à une situation de dépolarisation spontanée du ventricule gauche.

3. le dispositif de la revendication 1, dans lequel :

- les moyens (10, 12, 20, 30) de détection d'événements auriculaires et ventriculaires comprennent des moyens aptes à recueillir au cours d'un même cycle cardiaque, concurremment sur des voies respectives distinctes, au moins deux signaux différents d'électrogramme endocavitaire, EGM, et en dériver au moins deux composantes temporelles (*Vbip*, *Vuni*) distinctes respectives ; et

- lesdits moyens de quantification d'un degré de fusion comprennent des moyens d'analyse du cycle cardiaque courant, comportant :

   • des moyens (100) aptes à combiner les au moins deux composantes temporelles (*Vbip, Vuni*) en au moins une caractéristique 2D paramétrique (VGM) représentative dudit cycle cardiaque, à partir des variations de l'une des composantes temporelles en fonction de l'autre ; et
   
   • des moyens (106) aptes à opérer une comparaison de la caractéristique 2D du cycle courant avec au moins une caractéristique 2D de référence obtenue antérieurement et mémorisée par le dispositif.

4. le dispositif de la revendication 1, dans lequel lesdits moyens de quantification d'un degré de fusion sont aptes à calculer ladite valeur de taux de fusion sur un nombre donné C de cycles cardiaques, $C \geq 1$.

5. le dispositif de la revendication 3, dans lequel ledit nombre donné C de cycles cardiaques est un nombre variable, fonction d'un niveau d'activité du patient.

6. Le dispositif de la revendication 1, dans lequel les moyens d'adaptation dynamique du DAV (206, 208) comprennent des moyens de modification incrémentale du DAV (208), aptes à opérer :

   • une incrémentation d'au moins un pas du DAV lorsque la valeur courante du taux de fusion a évolué par rapport au(x) cycle(s) précédent(s) dans le sens d'un rapprochement vers ladite situation de capture complète, et
   
   • une décrémentation d'au moins un pas du DAV lorsque la valeur courante du taux de fusion a évolué par rapport au(x) cycle(s) précédent(s) dans le sens d'un rapprochement vers ladite situation de de contraction spontanée.

7. Le dispositif de la revendication 6, dans lequel ladite incrémentation/décrémentation est une incrémentation/décrémentation d'un nombre variable de pas, ledit nombre de pas étant fonction de l'écart entre la valeur courante et la valeur cible du taux de fusion.

8. Le dispositif de la revendication 1, comprenant en outre des moyens de comparaison (210, 212) de la valeur de DAV déterminée par les moyens d'adaptation dynamique du DAV (208) avec un seuil limite supérieur (DAV$_{max}$), et des moyens de modification sélective du mode de fonctionnement du dispositif (214) en cas de franchissement dudit seuil limite supérieur.

9. Le dispositif de la revendication 1, dans lequel les moyens d'adaptation dynamique du DAV comprennent des moyens de limitation (210, 216) de ladite valeur de DAV modifiée à une valeur plancher minimale (DAV$_{min}$).

10. Le dispositif de la revendication 1, comprenant en outre :

   - des moyens (10, 20) de stimulation auriculaire, sélectivement commutables entre un mode avec stimulation auriculaire (A) et un mode sans stimulation auriculaire (P) ;
   - des moyens de détection et de comptage (300, 302), dans un premier compteur (Compteur 1), des commutations entre modes avec et sans stimulation auriculaire ;
   - des moyens de détection et de comptage (306, 308), dans un second compteur (Compteur 2), des occurrences où la variation de la valeur courante du taux de fusion entre cycles consécutifs excède un seuil prédéterminé ; et
   - des moyens de modification sélective (312) du mode de fonctionnement du dispositif en cas de dépassement par le premier et le second compteur de valeurs de compte respectives prédéterminées.

11. Le dispositif de la revendication 1, comprenant en outre :

   - des moyens (10, 20) de stimulation auriculaire, sélectivement commutables entre un mode avec stimulation auriculaire (A) et un mode sans stimulation auriculaire (P) ;
   - des moyens de détection et de comptage (300, 302), dans un premier compteur (Compteur 1), des commutations entre modes avec et sans stimulation auriculaire ;
   - des moyens de détection et de comptage (306, 308), dans un second compteur, (Compteur 2)

des occurrences où la variation de la valeur courante du taux de fusion entre cycles consécutifs excède un seuil prédéterminé ; et

- des moyens de modification sélective (312) du mode de fonctionnement du dispositif en cas de dépassement par le premier compteur d'une première valeur de compte prédéterminée, alors que le second compteur reste au-dessous d'une seconde valeur de compte prédéterminée, et *vice versa.*

**Patentansprüche**

1. Implantierbare medizinische Vorrichtung vom Typ kardialer Resynchronisations-Schrittmacher, umfassend:

   - Mittel (10, 12, 30) zur Herzkammererregung, die dazu geeignet sind, Erregungsimpulse zu erzeugen, welche dazu bestimmt sind, zumindest der linken Herzkammer (36) eines Patienten zugeführt zu werden, welcher die Vorrichtung trägt;
   - Mittel (10, 12, 20, 30) zur Erfassung von Vorhof- und Kammerereignissen; und
   - Mittel zur Anwendung einer atrioventrikulären Verzögerung (AVV) zwischen einem erfassten oder erregten Vorhofereignis und der Abgabe eines Impulses zur Herzkammererregung,

   wobei die Vorrichtung **dadurch gekennzeichnet ist, dass** sie weiterhin Folgendes umfasst:

   - Mittel (202) zur Quantifizierung eines Ausmaßes der zeitlichen Übereinstimmung zwischen der Abgabe eines Erregungsimpulses an einen, linken oder rechten, Herzraum und einer spontanen Kontraktion eines anderen, gegenüberliegenden, also rechten beziehungsweise linken, Herzraums, wobei diese Mittel dazu geeignet sind, einen Wert des Grads der zeitlichen Übereinstimmung zu berechnen, der zwischen zwei Extremwerten liegt, welche einer Situation des vollständigen Abfangens beziehungsweise einer Situation der vollständigen Depolarisierung dieses Herzraums entsprechen; und
   - Mittel (206, 208) zur dynamischen Anpassung der AW, die dazu geeignet sind, den Wert der AW, welche auf die Abgabe des Impulses zur Herzkammererregung angewendet wird, in Abhängigkeit von einem Abgleich zu modifizieren, welcher zwischen i) dem laufenden Wert des Grades der zeitlichen Übereinstimmung, wie er von den Quantifizierungsmitteln berechnet wurde, und ii) einem Sollwert des Grades der zeitlichen Übereinstimmung durchgeführt wird.

2. Vorrichtung nach Anspruch 1, wobei es sich bei den Mitteln (202) zur Quantifizierung eines Ausmaß der zeitlichen Übereinstimmung um Mittel (202) zur Quantifizierung eines Ausmaßes der zeitlichen Übereinstimmung zwischen der Abgabe eines Erregungsimpulses an die linke Herzkammer und einer spontanen Kontraktion der rechten Herzkammer handelt, wobei diese Mittel dazu geeignet sind, einen Wert des Grads der zeitlichen Übereinstimmung zu berechnen, der zwischen zwei Extremwerten liegt, welche einer Situation des vollständigen Abfangens beziehungsweise einer Situation der vollständigen Depolarisierung der linken Herzkammer entsprechen.

3. Vorrichtung nach Anspruch 1, wobei:

   - die Mittel (10, 12, 20, 30) zur Erfassung von Vorhof- und Herzkammerereignissen Mittel umfassen, die dazu geeignet sind, im Verlauf ein- und desselben Herzzyklus zeitgleich auf jeweils gesonderten Wegen mindestens zwei verschiedene Signale einer elektrophysiologischen Untersuchung, EPU, aufzunehmen und daraus mindestens zwei jeweils gesonderte zeitliche Komponenten (*Vbip, Vuni*) abzuleiten; und
   - wobei die Mittel zur Quantifizierung eines Ausmaßes der zeitlichen Übereinstimmung Mittel zur Untersuchung des laufenden Herzzyklus umfassen, welche Folgendes aufweisen:

     • Mittel (100), die dazu geeignet sind, die mindestens zwei zeitlichen Komponenten (*Vbip, Vuni*), ausgehend von den Veränderungen bei einer der zeitlichen Komponenten in Abhängigkeit von der anderen, in mindestens einer parametergestützten 2D-Kennlinie (VGM) zu vereinigen, welche für den Herzzyklus repräsentativ ist; und
     • Mittel (106), die dazu geeignet sind, einen Abgleich der 2D-Kennlinie des laufenden Zyklus mit mindestens einer Referenz-2D-Kennlinie durchzuführen, welche im Vorfeld erstellt wurde und von der Vorrichtung gespeichert wurde.

4. Vorrichtung nach Anspruch 1, wobei die Mittel zur Quantifizierung eines Ausmaßes der zeitlichen Übereinstimmung dazu geeignet sind, die Berechnung des Wertes des Grads der zeitlichen Übereinstimmung an einer gegebenen Anzahl C von Herzzyklen vorzunehmen, $C \geq 1$.

5. Vorrichtung nach Anspruch 3, wobei die gegebene Anzahl an Herzzyklen C eine variable Anzahl ist, welche vom Aktivitätsniveau des Patienten abhängt.

6. Vorrichtung nach Anspruch 1, wobei die Mittel (206, 208) zur dynamischen Anpassung der AVV Mittel (208) zur schrittweisen Modifizierung der AVV umfassen, welche dazu geeignet sind, Folgendes durchzuführen:

    • eine schrittweise Erhöhung der AVV um mindestens einen Schritt, wenn der laufende Wert des Grads der zeitlichen Übereinstimmung sich gegenüber dem/den vorhergehenden Zyklus/Zyklen derart entwickelt hat, dass er sich der Situation des vollständigen Abfangens annähert, und
    • eine schrittweise Verringerung der AVV um mindestens einen Schritt, wenn der laufende Wert des Grads der zeitlichen Übereinstimmung sich gegenüber dem/den vorhergehenden Zyklus/Zyklen derart entwickelt hat, dass er sich der Situation einer spontanen Kontraktion annähert.

7. Vorrichtung nach Anspruch 6, wobei es sich bei der schrittweisen Erhöhung/Verringerung um eine schrittweise Erhöhung/Verringerung um eine variable Anzahl von Schritten handelt, wobei die Anzahl an Schritten von der Abweichung zwischen dem laufenden Wert und dem Sollwert des Grads der zeitlichen Übereinstimmung abhängt.

8. Vorrichtung nach Anspruch 1, wobei sie darüber hinaus Mittel (210, 212) zum Abgleich des Werts der AW, wie er von den Mitteln (208) zur dynamischen Anpassung der AVV bestimmt wurde, mit einem oberen Grenzwert ($AVV_{max}$) sowie Mittel umfasst, welche den Betriebsmodus der Vorrichtung (214) auf selektive Weise modifizieren, wenn der obere Grenzwert überschritten wird.

9. Vorrichtung nach Anspruch 1, wobei Mittel zur dynamischen Anpassung der AVV Mittel (210, 216) umfassen, welche den modifizierten AW-Wert auf eine Mindestwert (AWmin) begrenzen.

10. Vorrichtung nach Anspruch 1, die darüber hinaus Folgendes umfasst:

    - Mittel (10, 20) zur Vorhoferregung, die wahlweise zwischen einem Modus mit Vorhoferregung (A) und einem Modus ohne Vorhoferregung (P) geschaltet werden können;
    - Mittel (300, 302) zur Erfassung und Auszählung, in einem ersten Zählwerk (Zählwert 1), der Schaltvorgänge zwischen den Modi mit und ohne Vorhoferregung;
    - Mittel (306, 308) zur Erfassung und Auszählung, in einem zweiten Zählwerk (Zählwerk 2), von Fällen, in welchen die Veränderung des laufenden Werts des Grads der zeitlichen Übereinstimmung zwischen aufeinanderfolgenden Zyklen einen vorbestimmten Schwellenwert überschreitet; und
    - Mittel (312) zur wahlweisen Modifizierung des Betriebsmodus der Vorrichtung, wenn das erste und das zweite Zählwerk ihre jeweiligen vorbestimmten Zählwerte überschreiten.

11. Vorrichtung nach Anspruch 1, die darüber hinaus Folgendes umfasst:

    - Mittel (10, 20) zur Vorhoferregung, die wahlweise zwischen einem Modus mit Vorhoferregung (A) und einem Modus ohne Vorhoferregung (P) geschaltet werden können;
    - Mittel (300, 302) zur Erfassung und Auszählung, in einem ersten Zählwerk (Zählwert 1), der Schaltvorgänge zwischen den Modi mit und ohne Vorhoferregung;
    - Mittel (306, 308) zur Erfassung und Auszählung, in einem zweiten Zählwerk (Zählwerk 2), von Fällen, in welchen die Veränderung des laufenden Werts des Grads der zeitlichen Übereinstimmung zwischen aufeinanderfolgenden Zyklen einen vorbestimmten Schwellenwert überschreitet; und
    - Mittel (312) zur wahlweisen Modifizierung des Betriebsmodus der Vorrichtung, wenn das erste Zählwerk einen ersten vorbestimmten Zählwert überschreitet, während das zweite Zählwerk unterhalb eines zweiten vorbestimmten Zählwerts bleibt, und umgekehrt.

## Claims

1. An active implantable medical device of the cardiac resynchronizer type, comprising:

    - ventricular pacing means (10, 12, 30), suitable for generating pacing pulses to be delivered to at least the left ventricle (36) of a patient in whom the device is implanted;
    - means (10, 12, 20, 30) for detecting atrial and ventricular events; and
    - means for applying an atrioventricular delay, AVD, between a sensed or stimulated atrial event and delivery of a ventricular pacing pulse;

said device being **characterized in that** it further comprises:

    - quantification means (202) for quantifying a degree of fusion between the delivery of a pacing pulse to a cavity, left or right, and a spontaneous contraction of another, opposite, cavity, respectively right or left, these means being suitable for computing a fusion rate value lying between two extreme values corresponding respectively to a situation of full capture and to a situation of spontaneous depolarization of said cavity; and
- dynamic AVD adaptation means (206, 208) suitable for dynamically modifying the value of the AVD applied to delivery of said ventricular pacing pulse, as a function of a comparison made between i) the current value of the fusion rate computed by the quantification means, and ii) a target value for the fusion rate.

2. The device of claim 1, wherein the quantification means (202) for quantifying a degree of fusion are quantification means (202) for quantifying a degree of fusion between the delivery of a pacing pulse to the left ventricle and a spontaneous contraction of the right ventricle, these means being suitable for computing a fusion rate value lying between two extreme values corresponding respectively to a situation of full capture and to a situation of spontaneous depolarization of the left ventricle.

3. The device of claim 1, wherein:

   - the means (10, 12, 20, 30) for detecting atrial and ventricular events comprise means suitable for collecting at least two different endocardial electrogram, EGM, signals during the same cardiac cycle, concurrently on respective ones of distinct channels, and for deriving from said signals at least two respective distinct time components (V*bip,* V*uni*); and
   - said quantification means for quantifying a degree of fusion comprise analysis means for analyzing the current cardiac cycle, which analysis means comprise:

      • means (100) suitable for combining the at least two time components (V*bip,* V*uni*) into at least one parametric 2D characteristic curve (VGM) representative of said cardiac cycle, on the basis of the variations of one of the time components as a function of the other time component; and
      • means (106) suitable for comparing the 2D characteristic curve of the current cycle with at least one reference 2D characteristic curve obtained previously and stored in a memory by the device.

4. The device of claim 1, wherein said quantification means for quantifying a fusion rate are suitable for computing said fusion rate value over a given number C of cardiac cycles, where C ≥ 1.

5. The device of claim 3, wherein said given number C of cardiac cycles is a number that is variable, as a function of the level of activity of the patient.

6. The device of claim 1, wherein the dynamic AVD adaptation means (206, 208) comprise incremental AVD modification means (208) for incrementally modifying the AVD, which incremental AVD modification means are suitable for effecting:

   • an incrementation by at least one step of the AVD when the current value of the fusion rate has shifted relative to the preceding cycle(s) towards said situation of full capture; and
   • a decrementation by at least one step of the AVD when the current value of the fusion rate has shifted relative to the preceding cycle(s) towards said situation of spontaneous contraction.

7. The device of claim 6, wherein said incrementation/decrementation is an incrementation/decrementation by a variable number of steps, said number of steps being a function of the difference between the current value and the target value of the fusion rate.

8. The device of claim 1, further comprising comparison means (210, 212) for comparing the AVD value determined by the dynamic AVD adaptation means (208) with an upper limit threshold (AVD$_{max}$), and selective modification means for selectively modifying the operating mode of the device (214) in the event said upper limit is exceeded.

9. The device of claim 1, wherein the dynamic AVD adaptation means comprise limitation means (210, 216) for limiting said AVD value as modified to no lower than a minimum lower limit value (AVD$_{min}$).

10. The device of claim 1, further comprising:

    - atrial pacing means (10, 20) that are selectively switchable between a mode with atrial pacing (A) and a mode without atrial pacing (P);
    - detecting and counting means (300, 302) for detecting and counting, in a first counter (Counter 1) the switchovers between modes with and without atrial pacing;
    - detecting and counting means (306, 308) for detecting and counting, in a second counter (Counter 2), occurrences of when the variation in the current value of the fusion rate between consecutive cycles exceeds a predetermined threshold; and
    - selective modification means (312) for selectively modifying the operating mode of the device in the event the first and the second counters exceed respective predetermined count values.

11. The device of claim 1, further comprising:

- atrial pacing means (10, 20) that are selectively switchable between a mode with atrial pacing (A) and a mode without atrial pacing (P);

- detecting and counting means (300, 302) for detecting and counting, in a first counter (Counter 1) the switchovers between modes with and without atrial pacing;

- detecting and counting means (306, 308) for detecting and counting, in a second counter (Counter 2), occurrences of when the variation in the current value of the fusion rate between consecutive cycles exceeds a predetermined threshold; and

- selective modification means (312) for selectively modifying the operating mode of the device in the event the first counter exceeds a first predetermined count value, while the second counter remains below a second predetermined count value, and *vice versa.*

Fig.1

**Fig.2**

$V_{bip}(V)$

$V_{uni}(V)$

**Fig.3**

$V_{bip}(V)$

Capture (DAV=30ms)

Cycle à tester (DAV=170ms)

Rythme spontané

temps (points d'échantillonnage @ 128Hz)

$V_{uni}(V)$

Cycle à tester (DAV=170ms)

Capture (DAV=30ms)

Rythme spontané

temps (points d'échantillonnage @ 128Hz)

Fig.4

Fig.5

# Fig.6

# Fig.7

# Fig.8

# Fig.9

# Fig.10

116

120

124

102,104

| DAV court et $f_{stim}$>>rythme sinusal | DAV long et $f_{stim}$>>rythme sinusal | DAV long et $f_{stim}$<<rythme sinusal |
|---|---|---|

cycles en capture complète

cycles avec V spontané et A stimulée

cycles avec A et V spontanées

118

| Choix d'un cycle représentatif | Choix d'un cycle représentatif | Choix d'un cycle représentatif |
|---|---|---|

Référence n°1 (capture)

122

Référence n°2 (rythme spontané)

126

Référence n°3

non

| Référence n°1 et Référence n°2 suffisamment différentes ? | Détermination du décalage entre A stimulée et A spontanée |
|---|---|

128

Références invalides (trop proches) mettre à jour Ref.1

oui

130

Réf.1, Ref.2 validées

Détermination des distances entre Réf.1 , Ref.2 et
- les cycles en capture complète (→X1, X2)
- les cycles en rythme spontané (→Y1, Y2)

132

(X2-X1) et (Y1-Y2)

## Fig.11

$W_{DAV}$

$V_{bip}$

$V_{uni}$

DAV fictif

A

## Fig.12

W

R

$V_{bip}$

$V_{uni}$

R

A

# Fig.13

# Fig.14

**Fig.15**

# Fig.16

300

Commutation
A / P ?　　non

oui　　302

Incrémentation compteur 1

306

Compteur 1
> seuil ?　　non

oui

308

Variation
significative
du degré de
fusion ?　　non

oui　　308

Incrémentation compteur 2

310

Compteur 2
> seuil ?　　non

oui

Modification du mode
de fonctionnement
du stimulateur　　312

# Fig.17

306

Variation
significative
du degré de
fusion ?　　non

308　　oui

Incrémentation Compteur 2

310

Compteur 2
> seuil ?　　non

312　　oui

Modification du mode
de fonctionnement
du stimulateur

## RÉFÉRENCES CITÉES DANS LA DESCRIPTION

**Documents brevets cités dans la description**

- EP 2324885 A1
- EP 1205214 A1
- EP 2756865 A1

**Littérature non-brevet citée dans la description**

- **MILPIED et al.** Arrhythmia Discrimination in Implantable Cardioverter Defibrillators using Support Vector Machines applied to a new Représentation of Electrograms. *IEEE Transactions on Biomédical Engineering,* Juin 2011, vol. 58 (6), 1797-1803